# EUROPEAN PATENT SPECIFICATION

(11) **EP 1 551 441 B1**
(45) Date of publication and mention of the grant of the patent: **13.12.2006**
(21) Application number: 03758151.9
(22) Date of filing: 07.10.2003
(51) Int. Cl.: A61K 38/04, A61K 38/13, A61K 38/21

(54) **PEPTIDES AND RECOMBINANT PROTEINS MIMICKING INTERFERONS**
INTERFERON NACHAHMENDE PEPTIDE UND REKOMBINANTE PROTEINE
PEPTIDES ET PROTEINES RECOMBINANTES IMITANT LES INTERFERONS

(30) Priority: 07.10.2002 FI 20021774
(43) Date of publication of application: 13.07.2005
(73) Proprietor: Zav'yalov, Vladimir Petrovich, Moscow region, 142380 (RU); Dolgikh, Dmitriy Alexandrovich, Moscow 115093 (RU); Kirpichnikov, Mikhail Petrovich, Moscow, 123022 (RU); Cherikova, Rita Valerievna, Moscow 103460 (RU); Abdullaev, Ziedulla Hikmatullaevich, Tula 300045 (RU); Korpela, Timo Kalevi, 20610 Turku (FI)
(72) Inventor: Zav'yalov, Vladimir Petrovich, Moscow region, 142380 (RU); Dolgikh, Dmitriy Alexandrovich, Moscow 115093 (RU); Kirpichnikov, Mikhail Petrovich, Moscow, 123022 (RU); Cherikova, Rita Valerievna, Moscow 103460 (RU); Abdullaev, Ziedulla Hikmatullaevich, Tula 300045 (RU); Korpela, Timo Kalevi, 20610 Turku (FI)
(74) Representative: Karvinen, Leena Maria
(86) International application number: PCT/FI2003/000736
(87) International publication number: WO 2004/030686

(56) References cited:
- WO-A1-95/30759
- WO-A1-98/52594
- WO-A2-02/44197
- DATABASE MEDLINE [Online] CHERTKOVA R.V. ET AL.: 'Synthetic proteins with antiviral properties of alpha-interferons', XP002973671 Database accession no. (NLM12845801) & BIOORGRANICHESKAIA KHIMIIA vol. 29, no. 3, pages 258 - 268
- DATABASE CA [Online] CHERTKOVA R.V. ET AL.: 'Antiviral properties of de novo designed proteins based on the albeferon and LKDRHDF (30-36) fragment from human interferon-alpha 2', XP002973672 Retrieved from STN Database accession no. 138:82960 & BIOPOLIMERI I KLITINA vol. 18, no. 2, 2002, pages 161 - 163
- DOLGIKH DMITRY A. ET AL.: 'The de novo protein with grafted biological function: transferring of interferon blast-transforming activity to albebetin' PROTEIN ENGINEERING vol. 9, no. 2, 1996, pages 195 - 201, XP002973674
- DATABASE CA [Online] APHASIZHEVA I. ET AL.: 'Influence of a biologically active interferon fragment on the carrier de novo protein structure', XP002973673 Retrieved from STN Database accession no. 131:101083 & BIOFIZIKA vol. 43, no. 3, 1998, pages 384 - 391

## Description

### FIELD OF INVENTION

This invention is related to human or animal medicine, and more specifically to improving the therapy of antiviral and antiproliferative drugs whenever the effective ingredients may contain interferons. This invention provides novel peptides and recombinant proteins mimicking antiviral and antiproliferative activities of interferons, but not having their harmful side-effects.

### BACKGROUND OF INVENTION

The present invention is based on a profound computer modeling examination of the structures of interferons from different sources and identification of two bioactive sites of the interferons one of which is the part of another. Such data combined with biological studies with synthetic peptides led us to a surprising finding that the small artificial peptides specifically combined together can owe almost the all of the biological activity of the parent interferon. The main advantage of such artificial mini-interferon is the finding that many of the harmful side-effects of interferons can be avoided.

Interferons are the first cytokines produced by recombinant DNA technology (Taniguchi T. et al., 1980, Nature 285, pp. 547-549). The potent antiviral activity of interferons together with their potential antitumor (antiproliferative) actions provided the impetus for large-scale manufacture of interferons for the purpose of clinical evaluation in a variety of viral and malignant diseases (Pestka S. et al., 1987, Annual Review Biochem. 56, pp. 727-777; Meager A., 1998, In: Cytokines, Eds. Mire-Sluis A. and Thorpe R., Academic Press, pp. 361-389; Sen G.C., 2001, Annual Rev. Microbiol. 55, pp. 255-281; Bon A.L. and Tough D.F., 2002, Curr. Opin. Immun. 14, pp. 432-436).

Interferons are divided into two types. The type I interferons include the fibroblast or interferon-β, a number of subtypes of the leukocytes, or interferon-α, trofoblast or interferon-τ and interferon-ω. The type II interferon is represented by immune-interferon, or interferon-γ (De Maeyer E. and De Maeyer-Guignard J., 1998, In: Cytokines, Eds. Mire-Sluis A. and Thorpe R., Academic Press, pp. 392-400). The type I interferons are stable at pH 2 and realize their antiviral properties interacting with a common cell receptor, IFNAR. This receptor consists of two trans-membrane proteins: IFNAR1 and IFNAR2. Interferons-α bind first to IFNAR2, and then to IFNAR1. IFNAR2 is the main ligand-binding subunit of the receptor with K_{d} ≈ 2-10×10⁻⁹ M for human interferon-α₂. Affinity of IFNAR1 to human interferon-α₂ is essentially lower (K_{d} > 100×10⁻⁹ M), but its participation in the binding reaction increases affinity of the receptor to ligand approximately 10 times and is necessary for the induction of intracellular signal. Association of IFNAR1 and IFNAR2 into the receptor complex activates the IFNAR-associated cytoplasmic tyrosine-kinases (JAK1 and TYK2), which phosphorylate each other and the intracellular parts of the receptor subunits. It results, in turn, in phosphorylation and activation of latent cytoplasmic factors of transcription (STAT1 and STAT2). Together with third component (p48) the factors form complex, which activates transcription of the interferon-stimulated genes, ISG (Zav'yalov V. and Zav'yalova G., 1997, Acta Pathol. Microbiol. Immun. Scand. 105, pp. 161-186).

The three-dimensional structure of human interferon-α₂ was obtained by the methods of X-ray analysis (Radhakrishnan R. et al., 1996, Structure 4, pp. 1453-1463) and NMR-spectroscopy (Klaus W. et al., 1997, J. Mol. Biol. 274, pp. 661-675). Human interferon-α₂ belongs to the α-helical cytokines (Zav'yalov V. et al., 1990, Biochim. Biophys. Acta 1041, pp. 178-185). Its globular structure includes five α-helices. Studies on the sequential epitope mapping by monoclonal antibodies, synthetic peptides, natural and *de novo* mutants (Zav'yalov V. et al., 1990, Biochim. Biophys. Acta 1041, pp. 178-185; Fish E.N., 1992, J. Interferon Res. 12, pp. 257-266; Senda T. et al., 1995, J. Mol. Biol. 253, pp. 187-207; Piehler J. and Schreiber G., 1999, J. Mol. Biol. 294, pp. 223-237; see also reviews: Zav'yalov V. and Zav'yalova G., 1997, Acta Pathol. Microbiol. Immunol. Scand. 105, pp. 161-186; Mitsui Y. et al., 1993, Pharmac. Ther. 58, pp. 93-132; Uze G. et al., 1995, J. Interferon Cytokine Res. 15, pp. 3-26) showed that the sequences corresponding to the N-terminal half of the loop AB (residues 25-35), the N-terminal half of helix D, and the C-terminal part of the loop DE (residues 121-134) make the main contribution to forming of the IFNAR2 binding site as well as to antiviral and antiproliferative activities of human interferon-α₂. These sequences are close together in the 3D-model of human interferon-α₂ (Piehler J. and Schreiber G., 1999, J. Mol. Biol. 294, pp. 223-237). The residues Leu30 and Arg33 in the N-terminal part of this site are absolutely necessary for antiviral activity of human interferon-α₂ (in particular, substitution of Arg33 for Lys or Ala decreases activity by 500 and 2500 times, respectively. Their role in antiviral activity correlates with their contribution to the interaction with IFNAR2. The C-terminal part of the binding site is not so important for interaction with IFNAR2 and antiviral activity. At the same time synthetic peptides corresponding to the sequences 124-138 (Danilkovich A. et al., 1992, Immunol. Lett. 31, pp. 15-20; Danilkovich A. et al., 1995, FEBS Lett. 369, pp. 161-164) and 130-137 (LKEKKYSP, Zav'yalov V. et al., WO9852594) suppress proliferation of human T-lymphoblastoid cell line MT-4 at concentrations 10⁻⁹ *-* 10⁻⁸ M, but does not possess essential antiviral activity.

The sequence LKEKKYSP (130-137) human interferon-α₂ was inserted into the N-terminal part of *de novo* protein albebetin (Fedorov A. et al., 1992, J. Mol. Biol. 225, pp. 927-931). As a result the *de novo* protein albeferon with pre-designed structure and function (Dolgikh D.A. et al., 1993, Biophysics 38, pp. 59-66) was engineered. Study of its structure and activity showed that albeferon possessed more compact and stable structure than albebetin (Aphsizheva I. et al., 1998, FEBS Lett. 425/1, pp. 101-104), stimulated proliferation of mouse thymocytes at lower concentrations than human interferon-α₂ (Dolgikh D. et al., 1996, Protein Engineering 9, pp. 195-201), and efficiently suppressed proliferation of human T-lymphoblastoid cell line MT-4 at concentrations 10⁻⁹ - 10⁻⁸ M, that is comparable to antiproliferative activity of human interferon-α₂ (Zav'yalov V. et al., WO9852594).

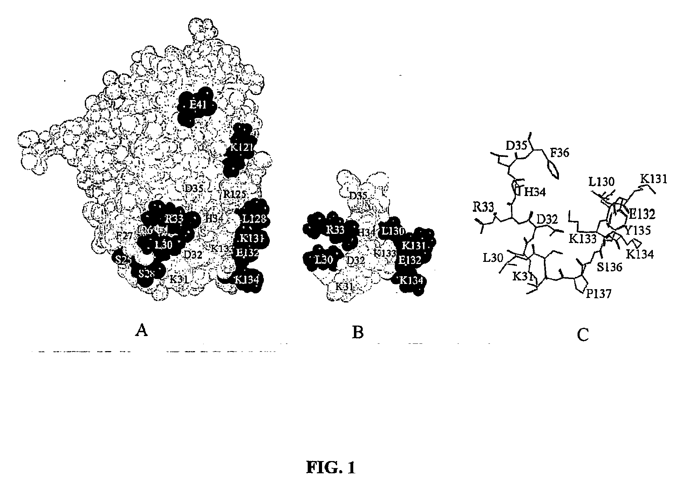

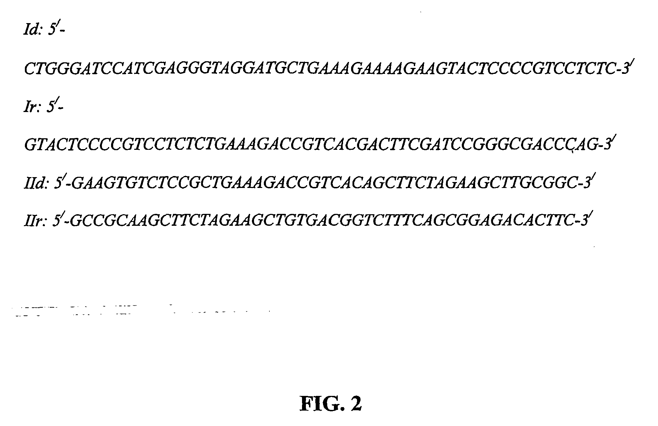

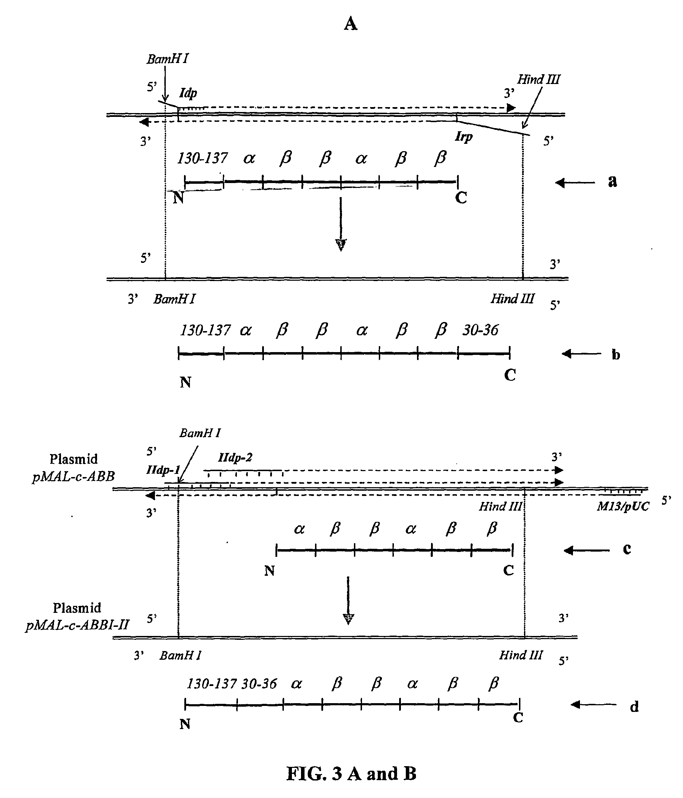

While it has been a common hope among scientist to produce artificial proteins and peptides having similar biological functions as interferons, nobody has managed to do that succesfully. One such attempt is described in the abstract publication by Chertkova, R.V., et al. in "Biopolimeri I Klitina", vol. 18, pp. 161-163 describes fusion of the fragment LKDRHDF (30-36) from human interferon-α2 (HuIFN-α2) to the C-terminus of albeferon de novo protein and also inserted into the N-terminus of albeferon just after the fragment LKEKKYSP (130-137) without any linker sequence between the peptides. The results of the study, presented in Figs 1 a and b in the cited document, show, in fact, low (if any) antiviral activity for said proteins, because the control proteins HuIFN-α2 and HuIFN-γ had considerably lower activity than IL-2-and IL-4. Besides that, IL-4 shows quite different activity in VERO and L-41 cells while all other proteins presented in the figure possess practically the same activity in both cell cultures. This indicates at least instability and unreliability of the results. Moreover, the cited document includes a serious internal contradiction: the English summary states that "both proteins prevented the destruction of the L-41 and VERO cell monolayers generated by cytopathical action of a virus with the efficiency of native HuIFN-alpha2 and did not possess cytotoxicological properties" while both Russian and Ukrainian summaries included just the statement that "both proteins possessed anti-viral activity and did not possess cytotoxicological properties". Therefore, it is not possible to make any conclusion based on the results in the cited reference. In it, the activities of the peptides were not also studied in a pure state (like in the present invention) but only as impure fusions to artificial proteins, which fusion proteins were made by the recombinant methods. When such fusion protein is folded, it may obtain wrong non-active conformations depending on the host strain and conditions of expressing and purifying the product. The key difference and the main embodiment of the present invention over Chertkova, R.V. et al. in the journal "Biopolimeri I Klitina", vol. 18, pp. 161-163 is the artificial link between the interferon fragments that allows them adopting the correct conformation and consequently, significantly improve the antiviral activities of the parent peptides and even the peptides linked together without a spacer.

The additional difference of the present invention over Chertkova, R.V., et al. in the "Biopolimeri I Klitina", vol. 18, pp. 161-163 is the demonstration of antiviral activity in the chemically synthesized peptides LKEKKYSP, LKDRHDF and LKEKKYSP-Ser-Ser-LKDRHDF which form exactly describable structures. If these peptides are linked improperly to proteins they may lose their biological activities indicated by the results in the paper by Chertkova et al.

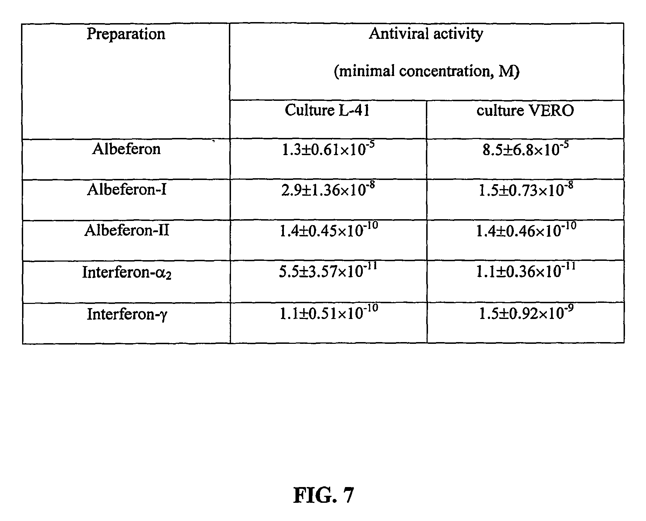

According to the present invention, the peptide LKEKKYSP-X-X-LKDRHDF (involving an artificial link between the peptides; X meaning a short side chain amino acid residue) was preferable and it practically completely reproduced the antiviral properties of HuIFN-α₂. We studied different peptide structures and we found surprisingly that these two fragments of HuIFN-α₂ locating very far from each other in the sequence synergize each other. However, they cannot adopt the correct structure related to their mutual position in the interferon's 3-D structure without an artificial link between the fragments (for example, two Ser residues or other two residues with a short side chain mimicking S-S bond Cys29-Cys138 in the HuIFNs-α molecules, see Fig. 1 of the present invention). Surprisingly, we found that only the fragments chemically connected with such link in peptide or in a fusion protein have the antiviral activity comparable with the HuIFN-α₂ (see Fig. 7 of the invention). The anti-viral activity of albeferon I, in which the fragments were inserted separately in the N- and C-termini, was in two orders lower than that of albeferon II. The anti-viral activity of albeferon, in which only the fragment LKEKKYSP (130-137) was inserted in the N-terminus, was in five orders lower than that of albeferon II.

During the recent years, an increasing attention is paid to the role of the type I interferons in immunity regulation (Bogdan C., 2000, Curr. Opin. Immunol. 12, pp. 419-424; Akbar A. et al., 2000, Immunol. Today 21, pp. 337-342; Sinigaglia F. et al., 1999, Immunol. Rev. 170, pp. 65-72). In particular, these interferons stimulate activity and differentiation of the antigen-activated Thl lymphocytes. They promote maturation of the most efficient antigen-presenting cells, i.e. the monocyte-originated dendritic cells and activated B-lymphocytes (Ruuth K. et al., 2001, Biochem. Biophys. Res. Commun. 284, pp. 583-586).

A major concern that has emerged from clinical studies of interferons is that type I interferons all generate a considerable number of undesirable, clinically observable, side effects. Nineteen % of patients suffering from autoimmune diseases, including autoimmune hepatitis, polymyositis, thyroiditis, rheumatoid arthritis, systemic lupus erythematosus (Ronnblom L. et al., 1991, Ann. Intern. Med. 115, pp. 178-183; Ioannou Y. and Isenberg D., 2000, Arthritis Rheum. 43, pp. 1431-1442; Ronnblom L. and Alm G., 2001, J. Exp. Med. 194, pp. F59-F63) obtain dangerous symptoms. In addition, a variable proportion (1-40%) of patients treated with human interferon-α or human interferon-β, especially recombinant human interferon-α₂ and recombinant human interferon-β Ser17, develop neutralizing antibodies to the interferon species used (Rinehart J. et al., 1986, Cancer Res. 46, pp. 5364-5367; Antonelli G. et al., 1991, J. Infect. Dis. 163, pp. 882-885), that in some instances have been associated with clinical "resistance" to interferon (Steis R. et al., 1988, N. Engl. J. Med. 318, pp. 1409-1413; Oberg K. et al., 1989, J. Natl. Cancer Inst. 81, pp. 531-535; Freund M. et al., 1989, Br. J. Haematol. 72, pp. 350-356; Fossa S. et al., 1992, Int. J. Cancer 50, pp. 868-870). Besides, interferons all generate fever, chills, malaise, myalgia, headache, fatigue, and weight loss, and in certain cases these have been severe enough for treatment to be halted. These clinical observations make very important engineering of the type I interferons mimetics specifically representing antiviral and antiproliferative activities of the proteins, devoid of sites responsible for the antibody response, of the induction of above-cited autoimmune diseases, and devoid of other undesirable side effects.

The present invention fulfils the above-described requirements for the development of novel peptides and proteins having specific biological activities of interferons without their side-effects during treatment of different diseases.

### SUMMARY OF INVENTION

The present invention provides peptide and recombinant protein mimetics specifically representing the antiviral and antiproliferative activities of interferons with avoiding interferons' undesirable side effects during treatment of a variety of diseases.

The basic embodiment of the present invention was to link together, through a spacer, two primary protein sequences locating distant to each other in the interferon's native structure. One of the two sequences, composing the antiviral site of interferons, corresponds to the antiproliferative site.

### BRIEF DESCRIPTION OF DRAWINGS

FIG.1 Tertiary structures of the human interferon-α₂ functional epitope responsible for its binding with IFNAR2 and antiviral activity (A) and the presupposed biologically active conformation of the peptide LKEKKYSP-Ser-Ser-LKDRHDF mimicking the human interferon-α₂ epitope (B and C). The tertiary structures are shown as space-filing (A, B) or stick (C) models based on the atomic coordinates of the human interferon-α₂ amino acid residues in solution (Klaus W. et al., 1997, J. Mol. Biol. 274, pp. 661-675). According to mutagenesis study (Piehler J. and Schreiber G., 1999, J. Mol. Biol. 294, pp. 223-237) the residues R33, L30 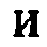 L26 play key role in interaction with IFNAR2 and antiviral activity; the residues F27, K31, D32, H34, D35, R125 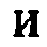 K133 are also important for these activities. On the contrary, the residues S25, S28, K121, L128, K131, E132 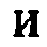 E134 make minimal contribution to the activities. The models were obtained using the program Chem-X (Chemical Design Ltd., UK)
FIG. 2 Structure of oligonucleotides used in PCR to construct the *de novo* proteins albeferon-I and albeferon-II genes. The restriction sites and the sequences corresponding to peptide-fragments LKEKKYSP and LKDRHDF are shown; overlapping sequences of primers IIdp-1 and IIdp-2 are shown by the double underlining; the sequence coding a cleavage site of protease "factor Xa" is shown by grey color.

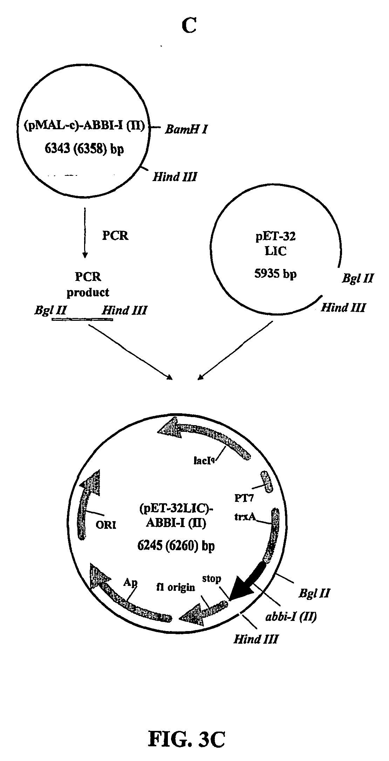

FIG. 3 Engineering of the *de novo* proteins albeferon-I (ABBI-I) and albeferon-II (ABBI-II) genes and expression plasmid vectors *pET-32 LIC* carrying these genes. (A) - scheme of PCR to engineer the albeferon-I (ABBI-I) gene using the albeferon (ABBI) gene as a matrix; (B) - scheme of PCR to engineer the albeferon-II (ABBI-II) gene using the albebetin (ABB) gene as a matrix; a,b,c,d - mutual positions of the regular secondary structure elements and the human interferon-α₂ sequences for albeferon (ABBI), albeferon-I (ABBI-I), albebetin (ABB), albeferon-II (ABBI-II), respectively; (C) - scheme of cloning of the albeferon-I (ABBI-I) and albeferon-II (ABBI-II) genes into a plasmid vector *pET-32 LIC.*

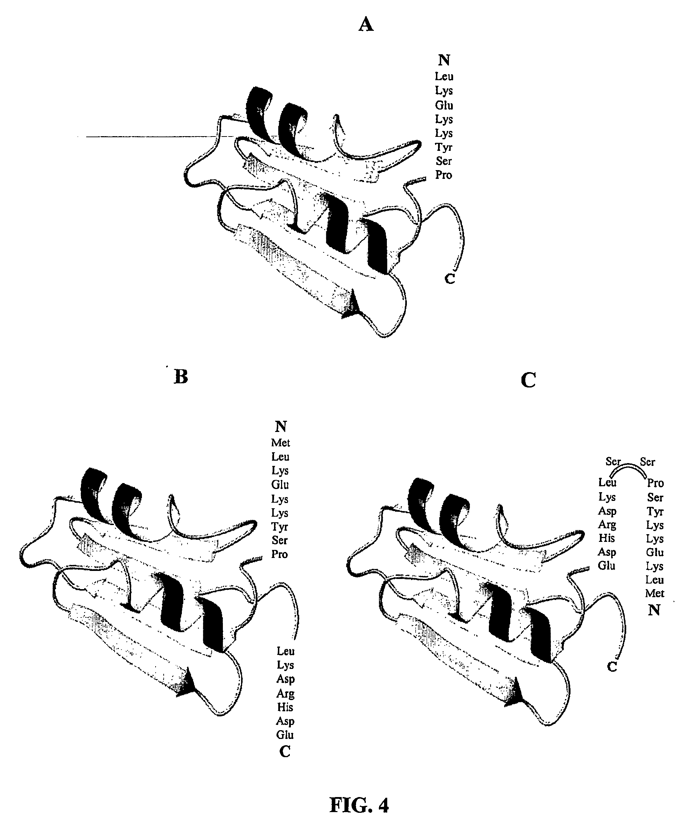

FIG. 4 Presupposed tertiary structures of *de novo* proteins albeferon (A), albeferon-I (B) 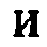 albeferon-II (C), including peptide sequences of human interferon-α₂. N- and C-terminal parts of the molecules and the human interferon-α₂ sequences are shown. Structures of the human interferon-α₂ sequences are shown conventionally.

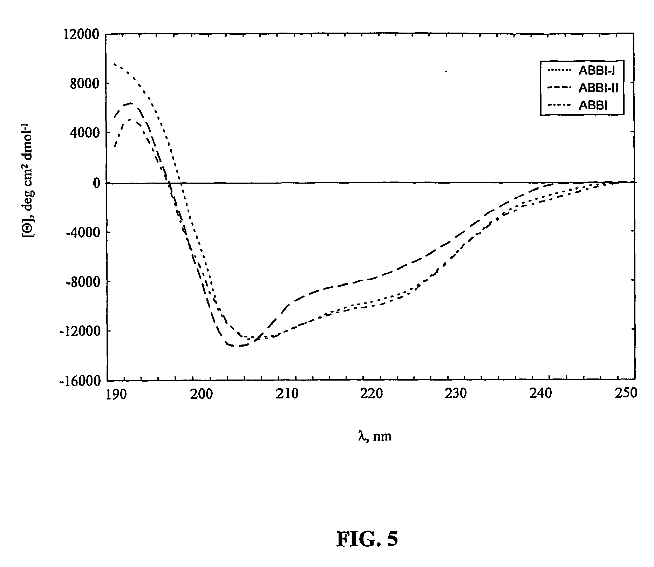

FIG. 5 CD-spectra of proteins albeferon (ABBI), albeferon-I (ABBI-I) and albeferon-II (ABBI-II) in far UV-region. Measurements were carried out in 20 mM NH₄HCO₃ buffer, pH 7.9 at 20°C. Concentration of proteins were 0.98 (albeferon), 0.96 (albeferon-I) and 1.04 (albeferon-II) mg/ml, cell pathlength was 1 mm.

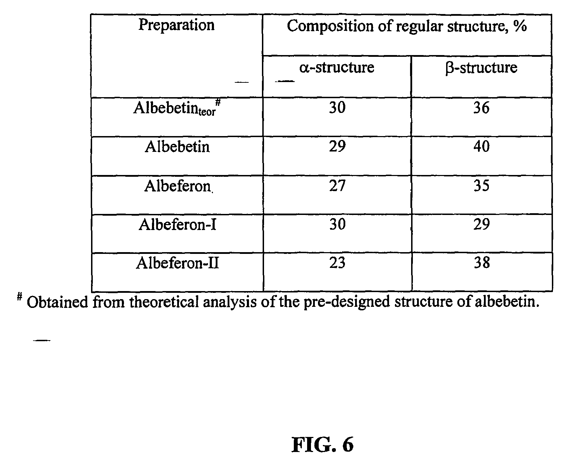

FIG. 6 Regular secondary structure of *de novo* proteins albebetin, albeferon, albeferon-I and albeferon-II.
FIG. 7 Antiviral properties of *de novo* proteins and interferons in cell cultures L-41 (human mononuclear leucosis) and VERO (kidney of green simian). The activity of preparations was determined as a minimal concentration leading to a 50 % delay of the virus of mouse encephalomiocarditis cytopathic action. The average values of three independent experiments are shown ± the standard deviations.

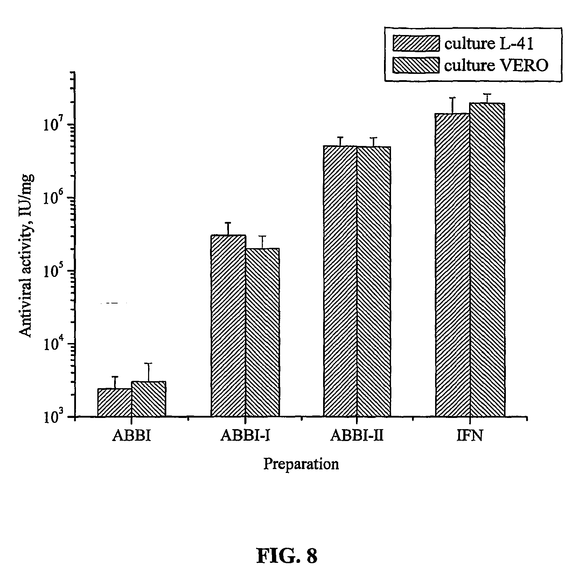

FIG. 8 Antiviral properties of *de novo* proteins in the cell cultures L-41 (human mononuclear leucosis) and VERO (kidney of green simian). Activities of proteins albeferon (ABBI), albeferon-I (ABBI-I), albeferon-II (ABBI-II) and recombinant human interferon-α₂ (IFN) were determined as minimal concentrations leading to a 50 % delay in cytopahtic action of the virus of mouse encephalomiocarditis. The average values of three independent experiments are shown ± standard deviations.

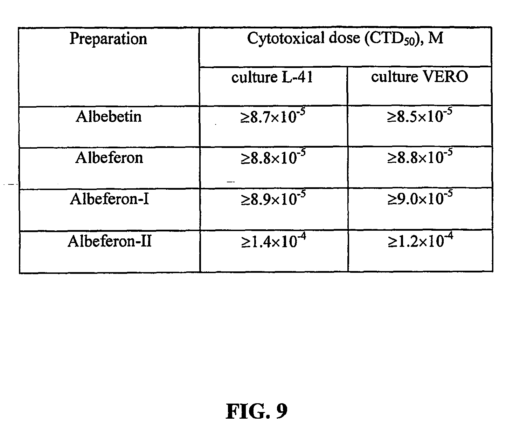

FIG. 9 Cytotoxical properties of *de novo* proteins albebetin, albeferon, albeferon-I and albeferon-II. Cytotoxical doses were determined as minimal concentrations leading to a 50 % destruction of a cell monolayer in absence of EMC virus.

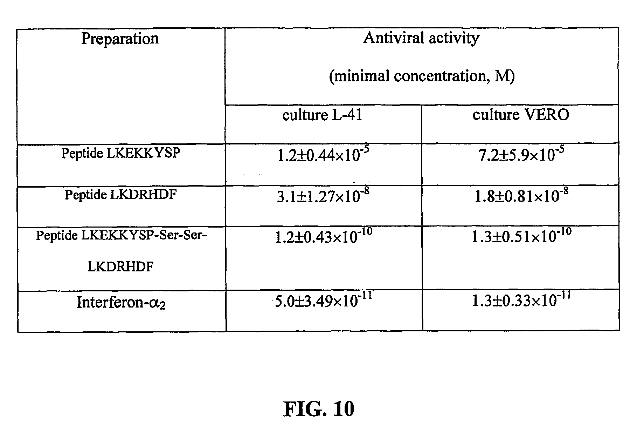

FIG. 10 Antiviral properties the synthetic linear peptide LKEKKYSP-Ser-Ser-LKDRHDF mimicking the human interferon-α₂ epitope in comperison with the recombinant human interferon-α₂ and the peptides LKEKKYSP and LKDRHDF, of *de novo* proteins in the cell cultures L-41 (human mononuclear leucosis) and VERO (kidney of green simian). Activities of peptides and recombinant human interferon-α₂ were determined as minimal concentrations leading to a 50 % delay in cytopahtic action of the virus of mouse encephalomiocarditis. The average values of three independent experiments are shown ± standard deviations.

### DETAILED DESCRIPTION OF THE INVENTION

The potent antiviral activity of type I interferons together with their potential antitumor (antiproliferative) actions provided the impetus for large-scale manufacture of interferons for the purpose of clinical evaluation in a variety of viral and malignant diseases. On the other hand, a major concern that emerged from clinical studies is that type I interferons all generate a considerable number of undesirable, clinically observable, side effects. It is most dangerous that 19 % patients suffer of autoimmune diseases including autoimmune hepatitis, polymyositis, thyroiditis, rheumatoid arthritis, systemic lupus erythematosus. In addition, a variable proportion (1-40%) of patients treated with human interferon-α or human interferon-β, especially recombinant human interferon-α₂ and recombinant human interferon-β Ser17, develop neutralizing antibodies to interferon species used, that in some instances have been associated with clinical "resistance" to interferon. Besides, type I interferons all generate fever, chills, malaise, myalgia, headache, fatigue, and weight loss, and in certain cases these have been severe enough for treatment to be halted. These clinical observations make very important engineering of the type I interferons mimetics specifically representing antiviral and antiproliferative activities of the proteins, but avoid of most sites responsible for undesirable side effects.

In the present invention, it was surprisingly found that the harmful side effects of interferons can be avoided by constructing artificial peptides or recombinant proteins carrying bioactive peptides. Such peptides were preliminarily identified by careful computer modeling analyses. The peptides were synthesized and randomly combined in different ways resulting in biologically very active artificial interferon mimetics. In particular, there was surprisingly observed that the artificial peptide LKEKKYSP-Ser-Ser-LKDRHDF, inserted into the N-termini of the *de novo* protein albebetin, demonstrated antiviral activity, being almost as active as human interferon-α₂. The protein revealed cytotoxicity only at concentrations exceeding the minimal active concentration by 6 orders of magnitude.

In the previous studies, there was found that synthetic peptide LKEKKYSP (α-peptoferon) suppresses proliferation of human T-lymphoblastoid cell line MT-4 at concentrations 10⁻⁹ - 10⁻⁸ M that is comparable to antiproliferative activity of recombinant human interferon-α₂ (Zav'yalov V. et al., WO9852594).

The sequence LKEKKYSP (130-137) human interferon-α₂ was inserted into the N-terminal part of *de novo* protein albebetin (Fedorov A. et al., 1992, J. Mol. Biol. 225, pp. 927-931). As a result the *de novo* protein albeferon with pre-designed structure and function was engineered (Dolgikh D.A. et al., 1993, Biophysics 38, pp. 59-66). Study of its structure and activity showed that albeferon possessed more compact and stable structure than albebetin (Aphsizheva I. et al., 1998, FEBS Lett. 425/1, pp. 101-104), and efficiently suppressed proliferation of human T-lymphoblastoid cell line MT-4 at concentrations 10⁻⁹ - 10⁻⁸ M, that is comparable to antiproliferative activity of recombinant human interferon-α₂ (Zav'yalov V. et al.,WO9852594).

It is well-known that all type I interferons compete for the common receptor IFNAR and can induce the common type I interferon activities. Therefore, it is reasonable to assume that in the process of natural selection the changes in the amino acid sequence of the binding/antiviral/antiproliferative site(s) of type I interferons were selected not to abolish the biological activities of the site. Consequently, all type I natural and recombinant interferons, as well as peptides corresponding to their binding/antiviral/antiproliferative site(s), and recombinant proteins having the amino acid sequences corresponding to the said site(s) might specifically represent the antiviral and antiproliferative activities.

For the testing of the peptide and recombinant protein mimetics specifically representing the binding/ativiral/antiproliferative site(s) of type I interferons, we employed the classic antiviral test systems with the cells of human mononuclear leucosis L-41 and green simian kidney VERO, as well as the classic anti-lymphoproliferative test systems with human peripheral polymorphonuclear cells and human T-lymphoblastoid cell line MT-4. The cell culture conditions and the conditions of the humoral cells in blood circulation are closely related. In fact, in cell cultures, which are commonly used for testing of potential drugs, the conditions are strictly maintained similar to the blood circulation as to the temperature, pH, buffer, minerals, CO₂ and O₂ partial pressures and, so on. On the other hand, in this special case the target cells of the peptide and recombinant protein mimetics specifically representing the binding/antiviral/antiproliferative site(s) of type I interferons being used as the drugs, exist specifically in the blood circulation in very equal conditions to the cell cultures. Thus, it is highly predictable that the peptides or recombinant proteins of the present invention can be used as medical drugs for purposes previously used for interferons as the active ingredient in the drug formulations.

The first embodiment of the present invention is that two totally separate peptide sequences, very far from each other in the polypeptide chain, can work synergistically if they are combined to each other properly by a linker of at least 1 amino acid residue. Although the linker sequence has preferably 2 amino acid residues, also longer linkers with possibly large side chains may be used, which residues restrict the free rotations around the linker. Equally well, other molecules, such as carbohydrates can be used as the linkers to bring the peptides together. In certain cases it is preferable that the active peptide is fused to a protein to increase its stability, solubility, targeting, or other properties. The key difference of the present invention over Chertkova, R.V. et al. in the journal "Biopolimeri I Klitina", vol. 18, pp. 161-163 is specifically the artificial link between the interferon's two peptide fragments that allows the fused peptide adopting the correct conformation and, consequently, maximally reproduce the antiviral activity of interferons. According to the present invention two interferon fragments can be joined together through an artificial link, typically by a peptide link, to get significantly more bioactive peptides than described by Chertkova et al. The additional difference of the present invention over Chertkova, R.V., et al. in the "Biopolimeri I Klitina", vol. 18, pp. 161-163 is herein the demonstration of antiviral activity of the chemically synthesized peptides LKEKKYSP, LKDRHDF and LKEKKYSP-Ser-Ser-LKDRHDF which form exactly describable structures free of possible interferences by the carrier proteins.

The peptides of length of at least 10 amino acid residues, either as such or fused to appropriate peptides or proteins, can be used for treatment of diseases such as malignant B-cell and chronic myelogenous leukemias, low-grade non-Hodgkin and cutaneous T cell lymphomas, carcinoid tumors, squamous epithelial tumours of the head and neck, primary, renal cell carcinoma, multiple and malignant melanomas, Kaposi sarcoma, and viral infectious diseases, such as chronic active hepatitis B and C, the diseases wherein interferons are known to be exploited.

Whereas the present invention describes definite structures of bioactive peptides with properties useful for applications as drugs, it is to be understood that such peptides or peptides having carrier molecules can be additionally modified by chemical or physical means. Such modifications can have typically the aim of prolonged release, increased bioavailability, or binding to a specific target cell or membrane.

The invention is further illustrated below by non-limiting examples.

### Example 1.

*Molecular modeling.* Molecular models of the human interferon-α₂ functional epitope structure and presupposed biologically active conformation of the mimicking peptide LKEKKYSP-Ser-Ser-LKDRHDF were obtained using the program Chem-X (Chemical Design Ltd., UK) and atomic coordinates of the corresponding amino acid residues in the human interferon-α₂ molecule in solution (Klaus W. et al., 1997, J. Mol. Biol. 274, pp. 661-675) (FIG. 1).

*Synthesis of peptides.* The peptides LKEKKYSP-Ser-Ser-LKDRHDF, LKEKKYSP and LKDRHDF were synthesized using pentafluorophenyl ethers of *N*-replaced amino acids by the solid-phase technique (430-A synthesizer, Applied Biosystems, USA).

The crude products were purified by HPLC on a chromatograph (Gilson, France) using a Zorbax ODS column (4x 150 mm, 5 µm, DuPont, USA) using linear gradient of water acetonitrile (95%) in 0.2% trichloroacetic acid (10-25%, 20 min) at a flow rate of 1 ml/min. According to the optical data at 220 nm, the content of the bulk substance was found to be 99%. The molecular mass of peptides was estimated by mass spectromic analysis using Vision 2000 spectrometer ("Thermo Bioanalysis", UK). The peptide structure was confirmed by the amino acid analysis on a D500 amino acid analyzer (Durrum, USA).

*Recombinant DNA constructions for expression of proteins genes albeferon-I and albeferon-II.* Gene of the *de novo* protein albebetin was engineered earlier (Fedorov A. et al., 1992, J. Mol. Biol. 225, pp. 927-931). Then functional *de novo* protein albeferon was obtained (Dolgikh D.A. et al., 1993, Biophysics 38, pp. 59-66) by introducing a fragment (130-LKEKKYSP-137) from human interferon-α₂ into the N-terminus of albebetin by means of polymerase chain reaction. Genes of these proteins were inserted into the plasmid expression vector *pMal-c* (New England BioLabs, USA) by restriction sites *BamHI* and *HindIII* (albebetin) and *EcoRI* and *HindIII* (albeferon). In the present work we engineered two new biologically active *de novo* proteins based on albebetin and albeferon using polymerasw chain reaction with synthesized oligonucleotide primers corresponding to the active peptide fragments (FIG. 2). The new protein albeferon-I was obtained by introducing an active fragment (30-LKDRHDF-36) from human interferon-α₂ into the C-terminus of albeferon. The polymerase chain reaction scheme is shown in FIG. 3A. Plasmid vector *pMal-c* carrying the albeferon gene was used as a matrix; the direct primer *Idp* included the restriction site *BamH I,* the reverse primer *Irp* included *Hind III* and the active fragment coding sequence. To construct a gene of another protein albeferon-II including both fragments (connected by two serin residues) in the N-terminal part of albeferon, two direct overlapping primers (*IIdp-1* and *IIdp-2*) (FIG. 2) were synthesized. The primer *IIdp-1* included the restriction sites *BamH I* and the sequences coding the protease "factor Xa" cleavage site and the fragment LKEKKYSP-S-S; the primer *IIdp-2* included part of the sequence of *IIdp-1* and the sequence corresponding to the second fragment LKDRHDF. The sequencing primer M13/pUC (New England Biolabs, CIIIA) was used as a reverse primer and the plasmid vector *pMal-c* carrying the albebetin gene was used as a matrix in polymerase chain reaction (FIG. 3B). The polymerase chain reaction products were treated with endonucleases *BamH I* and *Hind III* and cloned into the plasmid vector *pMal-c* by these restriction sites.

The *pMal-c* vector allows efficient expression of target proteins as fusion proteins with maltose binding protein following by their digestion by protease factor Xa. However the final yield of the target *de novo* proteins in this system was not sufficient because of a small fraction of *de novo* proteins (~9 kDa) in the fusion proteins (~54 kDa). Therefore we decided to use another fusion system based on the plasmid vector *pET-32 LIC* (Novagen, USA). This system includes a relatively small thioredoxin protein from *Escherichia coli* and gives a higher yield of the targets and a convenient scheme of their isolation and purification. Earlier this vector was used for efficient biosynthesis of albebetin carrying a fragment of differentiation factor HLDF (Chertkova R.V. et al., 2002, Bioorgan. Chemistry, Moscow, 28, in press). The vector contains the thioredoxin protein gene under control of strong promoter of bacteriophage T7 and includes the 6His-tag for efficient isolation and purification of hybrid protein using the immobilized-metal affinity chromatography on nikelnitrilotriacetat agarose. The expression level of fusion proteins in this construction is sufficiently high reaching up to 30-40% of a total cell protein (Chertkova R.V. et al., 2002, Bioorgan. Chemistry, Moscow, 28, in press).

To clone the *de novo* proteins genes into the plasmid *pET-32 LIC* the restriction site *Bgl II* was introduced into both genes by polymerase chain reaction. The direct primer *dp-pET* including *Bgl II* was synthesized; the sequencing primer M13/pUC was used as a reverse primer, and the plasmid *pMal-c* with the respective gene was used as a matrix. The polymerase chain reaction products were treated with endonucleases *Bgl II* and *Hind III* and were cloned into *pET-32 LIC* using te above sites (FIG. 3C). The obtained plasmid constructs were verified by sequencing and used for transformation of *E. coli.* Thus, two new proteins albeferon-I and albeferon-II with the peptide fragment LKDRHDF (30-36) from a consensus sequence of human interferon-α₂ were obtained. Their supposed tertiary structures are shown in FIGs. 4B and 4C.

*Expression of recombinant genes. De novo* proteins albebetin, albeferon, albeferon-I and albeferon-II were expressed as fusion proteins with thioredoxin in the *E. coli* strain BL21(DE3)*pLysS* as it was described earlier for a albebetin including a fragment of differentiation factor HLDF (Chertkova R.V. et al., 2002, Bioorgan. Chemistry, Moscow, 28, in press). The maximal yield of the fusion proteins (up to 30-40% from a total cell protein according to SDS-PAGE) was reached after a 8-9 h growth in TB-broth using a 0.6-1.0 mM concentration of inductor β-D-thiogalactopyranoside and 37°C.

*Isolation and purification of proteins. De novo* proteins expressed as fusion proteins with thioredoxin were purified using ion-exchange chromatography and then affinity chromatography on nikelnitrilotriacetat agarose. The obtained fusion proteins were digested with high-specific protease "factor Xa" and the reaction mixture was applied on a column with nikelnitrilotriacetat agarose (Chertkova R.V. et al., 2002, Bioorgan. Chemistry, Moscow, 28, in press). Final purification on the anion-exchange column with Mono Q HR (Pharmacia Biotech) resulted in >90 % purity according to SDS-PAGE. Molecular masses of the obtained proteins determined by mass-spectrometry corresponded to those calculated theoretically and were equal to 9.3 and 9.8 kDa for albeferon-I and albeferon-II, respectively. Homogeneity of obtained preparations was confirmed by SDS-PAGE analysis and also by the N-terminal amino acid sequencing. Nine N-terminal amino acid residues Met-Leu-Lys-Glu-Lys-Lys-Tyr-Ser-Pro were confirmed for both proteins. The elaborated procedure of isolation and purification of *de novo* proteins gave up to 12 mg/l of cell suspension.

*CD-spectra of de novo proteins.* The circular dichroism spectra of albeferon, albeferon-I and albeferon-II in far UV-region are shown in FIG. 5. From the data one can conclude that the proteins possesses similar spectra, typical for proteins with considerable regular secondary structure. Analysis of the spectra by the method of Provencher and Glocker (Provencher S. and Glocker J., 1981, Biochemistry 20, pp. 33) showed that albeferon-I and albeferon-II contains 30 and 23 % α- and 29 and 38% β-structure, respectively, and albeferon has 27% α- and 35% β-structure (Aphsizheva I. et al., 1998, FEBS Lett. 425/1, pp. 101-104) (FIG. 6). The obtained data are in good agreement with experimental data (29% α- and 40% β-structure) and theoretical calculation (30% α- and 36% β-structure) for the original protein albebetin (Dolgikh D. et al., 1996, Protein Engineering 9, pp. 195-201). Thus, introduction of the second human interferon-α fragment into the albeferon does not influence essentially on its secondary structure.

*Antiviral and cytotoxity properties of obtained proteins.* Testing of antiviral activity of the proteins was carried out *in vitro* monitoring the ability of the preparation to suppress cytopathic action of the test-virus (Ershov F., 1996, Interferon system: norm and pathology, Moscow, Medicine). Two cell lines were used in expirements: cells of human mononuclear leucosis L-41 and green simian kidney VERO. The virus of mouse encephalomiocarditis, possessing short circle of reproduction and high sensitivity to the action of interferons was choosen as a test-virus. Recombinant human interferon-α₂ and recombinant human interferon-γ were choosen as a positive control of antiviral activity. The results of testing are presented in FIG. 7. Both *de novo* proteins possess antiviral activity. In the case of albeferon-II (the protein including the artificial sequence LKEKKYSP-Ser-Ser-LKDRHDF in the N-terminal part of the molecule, see FIG. 4C) the antiviral activity is only one order of magnitude less then that of human interferon-α₂ and comparable to that of human interferon-γ. The activity of preparation of recombinant human interferon-α₂, albeferon, albeferon-I and albeferon-II in international units, was calculated using human leukocyte interferon-α₂ as interferons standard (FIG. 8). It should be noted that the initial protein albeferon also possessed weak antiviral properties.

Cytotoxical doses (CTD₅₀) of the proteins were determined as their concentrations which cause 50 % cell destruction after 24 h of contact with cells in absent of virus (Chidjov N. et al., 1988, Basics of experimental chemotherapy of viral infections, Riga, Zinatne). It was showed, that all tested *de novo* proteins revealed cytotoxical properties only at comparatively high concentrations, exceed 8x10⁻⁵ M (FIG. 9). In particular, the cytotoxicity of albeferon-II was observed at a concentration ~10⁶ higher then that necessary for antiviral action of this protein.

Thus, we engineered and obtained two *de novo* proteins, one of which (albeferon-II) practically completely reproduces antiviral properties of human interferon-α₂. The protein albeferon-II including the artificial sequence LKEKKYSP-Ser-Ser-LKDRHDF in its N-terminal part possessed -two order of magnitude higher antiviral activity then albeferon-I, in which the sequences LKEKKYSP and LKDRHDF are on the opposite termini of the molecule. One can suppose that the opposite positions of the sequences may result in some steric or/and kinetic limitation for its unification in single antiviral center. This supposition is confirmed by an experimental evidence on the influence of the Cys29-Cys139 disulfide bond on the biological activity of type I interferons. This bond connecting N-terminal part of loop AB and N-terminal part of helix E in all interferons-α, is absent in mouse interferon-β. Mutant mouse interferon-β, in which the disulfide bond was introduced by genetic engineering, possesses ~10 times higher antiviral activity than the wild type protein (Day C. et al., 1992, J. Interferon Res. 12, pp. 139-143). In albeferon-II this disulfide bond was substituted by two serin residues connected by a peptide bond. The X-ray analysis showed that in crystal of human interferon-α₂ the Oδ1 and Oδ2 side chain atoms of Asp32 are between Nζ groups of Lys31 and Lys133 like in a sandwich (Radhakrishnan R. et al., 1996, Structure 4, pp. 1453-1463). These interactions could stabilize biologically active conformation of the artificial sequence LKEKKYSP-Ser-Ser-LKDRHDF of albeferon-II when the complex with IFNAR2 is formed.

The obtained date evidence that both human interferon-α₂ fragments LKEKKYSP (130-137) and LKDRHDF (30-36) contribute in formation of the antiviral center, however, the contribution of the fragment LKDRHDF is dominated. This conclusion completely coincides with the data of mutagenesis (Piehler J. and Schreiber G., 1999, J. Mol. Biol. 294, pp. 223-237).

### Example 2.

*Antiviral properties of synthetic linear peptides.* Testing of antiviral activity of the synthetic liear peptides was carried out *in vitro* monitoring the ability of the preparation to suppress cytopathic action of the test-virus (Ershov F., 1996, Interferon system: norm and pathology, Moscow, Medicine). Two cell lines were used in expirements: cells of human mononuclear leucosis L-41 and green simian kidney VERO. The virus of mouse encephalomiocarditis possessing short circle of reproduction and high sensitivity to the action of interfgerons was choosen as a test-virus. Human recombinant human interferon-α₂ was choosen as a positive control of antiviral activity. The results of testing are presented in FIG. 10. The antiviral activity of the peptide LKEKKYSP-Ser-Ser-LKDRHDF is only one order of magnitude less than that of human interferon-α₂.

Thus, the linear synthetic peptide LKEKKYSP-Ser-Ser-LKDRHDF practically completely reproduces antiviral properties of human interferon-α₂.

## Claims

1. A fusion peptide sequence, composed of two peptides from interferons bound together with a linker of two amino acid residues, wherein the fusion peptide sequence is selected from the group consisting of the peptide described in the top-horizontal line sequence of the following table and peptides containing at least 10 amino acid residues which are identical with those in the top-horizontal line sequence, wherein each amino acid residue of the top sequence may be substituted with any of the amino acid residues shown in the vertical line under it:
| L | T | E | K | K | Y | S | P | S | S | L | K | D | R | H | D | F |
|---|---|---|---|---|---|---|---|---|---|---|---|---|---|---|---|---|
| | R | R | R | R | H | R | R | A | A | | Q | A | K | N | E | |
| | Q | D | N | D | | D | D | T | T | | E | Y | | M | N | |
| | M | L | M | N | | N | L | G | G | | T | | | K | Y | |
| | I | S | | S | | | S | | | | M | | | Q | | |
| | K | K | | G | | | H | | | | | | | A | | |
| | E | A | | E | | | A | | | | | | | | | |
| | | G | | I | | | | | | | | | | | | |

2. A fusion peptide sequence according to Claim 1, wherein the linker sequence is Ser-Ser.

3. A drug composition comprising one or more of the fusion peptide sequences according to Claim 1.

4. A recombinant protein comprising one or more of the peptide sequences according to Claim 1 fused to a recombinant carrier protein.

5. A recombinant protein according to Claim 4, wherein the carrier protein is albebetin, serum albumin, or immunoglobulin G.

6. A drug composition comprising one or more of the recombinant proteins according to Claim 4.

## Patentansprüche

1. Fusionspeptidsequenz, zusammengesetzt aus zwei über einen Linker aus zwei Aminosäureresten miteinander verbundenen Interferonpeptiden, wobei die Fusionspeptidsequenz ausgewählt ist aus der Gruppe bestehend aus dem in der Sequenz der obersten horizontalen Zeile der nachstehenden Tabelle beschriebenen Peptid und Peptiden, die mindestens 10 Aminosäurereste enthalten, welche identisch sind mit der Sequenz der obersten horizontalen Zeile, wobei jeder Aminosäurerest der Sequenz der obersten horizontalen Zeile mit jedem der Aminosäurereste in der darunter gezeigten vertikalen Reihe ersetzt werden kann:
| L | T | E | K | K | Y | S | P | S | S | L | K | D | R | H | D | F |
|---|---|---|---|---|---|---|---|---|---|---|---|---|---|---|---|---|
| | R | R | R | R | H | R | R | A | A | | Q | A | K | N | E | |
| | Q | D | N | D | | D | D | T | T | | E | Y | | M | N | |
| | M | L | M | N | | N | L | G | G | | T | | | K | Y | |
| | I | S | | S | | | S | | | | M | | | Q | | |
| | K | K | | G | | | H | | | | | | | A | | |
| | E | A | | E | | | A | | | | | | | | | |
| | | G | | I | | | | | | | | | | | | |

2. Fusionpeptidsequenz nach Anspruch 1, wobei die Linkersequenz Ser-Ser ist.

3. Arzneistoffzusammensetzung, umfassend eine oder mehrere der Fusionspeptidsequenzen nach Anspruch 1.

4. Rekombinantes Protein, umfassend eine oder mehrere der Peptidsequenzen nach Anspruch 1, fusioniert mit einem rekombinanten Trägerprotein.

5. Rekombinantes Protein nach Anspruch 4, wobei das Trägerprotein Albebetin, Serumalbumin oder Immunglobulin G ist.

6. Arzneistoffzusammensetzung, umfassend eines oder mehrere der rekombinanten Proteine nach Anspruch 4.

## Revendications

1. Séquence de peptides de fusion, composée de deux peptides d'interférons liés avec un lieur de deux résidus d'acides aminés, dans laquelle la séquence de peptides de fusion est sélectionnée dans le groupe constitué du peptide décrit dans la séquence de la ligne horizontale supérieure du tableau suivant et des peptides contenant au moins 10 résidus d'acides aminés qui sont identiques à ceux de la séquence de la ligne horizontale supérieure, dans laquelle chaque résidu d'acide aminé de la séquence supérieure peut être substitué par l'un quelconque des résidus d'acide aminé présentés dans la ligne verticale sous celui-ci :
| L | T | E | K | K | Y | S | P | S | S | L | K | D | R | H | D | F |
|---|---|---|---|---|---|---|---|---|---|---|---|---|---|---|---|---|
| | R | R | R | R | H | R | R | A | A | | Q | A | K | N | E | |
| | Q | D | N | D | | D | D | T | T | | E | Y | | M | N | |
| | M | L | M | N | | N | L | G | G | | T | | | K | Y | |
| | I | S | | S | | | S | | | | M | | | Q | | |
| | K | K | | G | | | H | | | | | | | A | | |
| | E | A | | E | | | A | | | | | | | | | |
| | | G | | I | | | | | | | | | | | | |

2. Séquence de peptides de fusion selon la revendication 1, dans laquelle la séquence du lieur est Ser-Ser.

3. Composition de médicament comprenant une séquence ou plus de peptides de fusion selon la revendication 1.

4. Protéine recombinante comprenant une séquence ou plus de peptides selon la revendication 1 fusionnée à une protéine support recombinante.

5. Protéine recombinante selon la revendication 4, dans laquelle la protéine support est l'albébétine, la sérumalbumine ou l'immunoglobuline G.

6. Composition de médicament comprenant une des protéines recombinantes ou plus selon la revendication 4.
